# EUROPEAN PATENT APPLICATION

(11) **EP 4 378 904 A1**
(43) Date of publication of application: **05.06.2024**
(21) Application number: 22849187.4
(22) Date of filing: 06.07.2022
(51) Int. Cl.: C03C 12/00, A61K 6/836, C03C 3/062, C03C 3/064, C03C 3/083, C03C 3/091

(54) **X-RAY OPAQUE GLASS, GLASS FILLER, AND RESIN COMPOSITION**

(30) Priority: 29.07.2021 JP 2021124133
(71) Applicant: Nippon Electric Glass Co., Ltd., Otsu-shi, Shiga 520-8639 (JP)
(72) Inventor: ANDO, Tamio, Otsu-shi Shiga 520-8639 (JP); FUJITA, Shunsuke, Otsu-shi Shiga 520-8639 (JP)
(74) Representative: Henkel & Partner mbB
(86) International application number: PCT/JP2022/026820
(87) International publication number: WO 2023/008122

(57) **Abstract**

The purpose of the present invention is to provide a glass that can achieve a desired light transmission property and X-ray opacity when blended with a resin. Thus, provided is an X-ray opaque glass containing, by mass%, from 25 to 75% of SiO₂, from 0 to 15% of B₂O₃, from 5 to 23% of Al₂O₃, from 0 to 15% of K₂O, and from 0 to 30% of Cs₂O, and substantially not containing a Pb component. In the X-ray opaque glass, an aluminum equivalent thickness, which is an indicator of X-ray opacity, and a refractive index nd satisfy a relationship of (aluminum equivalent thickness)/(refractive index nd) ≥ 1.6.

## Description

### TECHNICAL FIELD

The present invention relates to an X-ray opaque glass suitable as a dental material, and also to a glass filler and a resin composition.

### BACKGROUND ART

A dental resin composition, which is a mixture of a resin and an inorganic filler, has been conventionally used for applications such as a dental restorative material, a denture base, a crown, and a temporary tooth. Usually, a UV-curable resin is used in dental resin compositions. For example, in the case of a dental restorative material, treatment is performed by applying a dental resin composition to a treatment site on a tooth and then irradiating the dental resin composition with UV light to cure the dental resin composition. Here, from the viewpoint of improving the aesthetic properties of teeth after treatment, a glass filler with high light transmission property has been proposed as the inorganic filler (see, for example, Patent Document 1)

Further, the glass filler is required to have X-ray opacity so that a treatment site of a tooth can be confirmed when an X-ray photograph is captured.

### CITATION LIST

### PATENT LITERATURE

Patent Document 1: JP 2010-202560 A

### SUMMARY OF INVENTION

### TECHNICAL PROBLEM

An effective measure for increasing the X-ray opacity of a glass filler is to include an element having a large atomic weight such as Ba or La in the glass composition. However, when these elements are included in the glass composition, the refractive index of the glass filler tends to increase. As a result, the difference in the refractive index from that of the resin matrix increases, and the light transmission property of the resin composition decreases, which may impair the aesthetic properties of the tooth after treatment.

In view of the above, an object of the present invention is to provide a glass by which a desired light transmission property and X-ray opacity can be achieved when mixed with a resin.

### SOLUTION TO PROBLEM

As a result of intensive studies, the present inventors discovered that the above problems can be solved by a glass having a specific composition and properties.

Namely, an X-ray opaque glass of the present invention contains, by mass%, from 25 to 75% of SiO₂, from 0 to 15% of B₂O₃, from 5 to 23% of Al₂O₃, from 0 to 15% of K₂O, and from 0 to 30% of Cs₂O, and substantially does not contain a Pb component, and is characterized in that an aluminum equivalent thickness, which is an indicator of X-ray opacity, and a refractive index nd satisfy a relationship of (aluminum equivalent thickness)/(refractive index nd) ≥ 1.6.

An X-ray opaque glass of the present invention is characterized by containing, by mass%, from 25 to 75% of SiO₂, from 0 to 15% of B₂O₃, from 5 to 23% of Al₂O₃, from 0 to 15% of K₂O, from 0 to 30% of Cs₂O, and from 10 to 40% of Cs₂O + BaO + SnO₂ + La₂O₃, and substantially not containing a Pb component.

The X-ray opaque glass of the present invention preferably has a refractive index nd of from 1.48 to 1.58.

The X-ray opaque glass of the present invention preferably has an X-ray opacity of 2.5 mm or more in terms of aluminum equivalent thickness.

Preferably, the X-ray opaque glass of the present invention substantially does not contain an F component.

The X-ray opaque glass of the present invention preferably contains, by mass%, 10% or more of Cs₂O + BaO + SnO₂ + La₂O₃. Note that "x + y + ..." as used herein refers to a total amount of the respective components.

Preferably, the X-ray opaque glass of the present invention substantially does not contain TiO₂.

The X-ray opaque glass of the present invention preferably contains, by mass%, from 0 to 6% of WO₃.

A glass filler of the present invention is characterized by being made of the X-ray opaque glass described above.

The glass filler of the present invention is preferably substantially spherical.

The glass filler of the present invention preferably has an average particle size of from 0.01 to 50 µm.

The surface of the glass filler of the present invention is preferably silanized.

A resin composition of the present invention is characterized by containing the glass filler described above and a curable resin.

The resin composition of the present invention preferably contains, by weight%, from 1 to 90% of the glass filler.

The resin composition of the present invention is preferably for dental use.

### ADVANTAGEOUS EFFECTS OF INVENTION

According to the present invention, a glass that can achieve a desired light transmission property and X-ray opacity when blended with a resin can be provided.

### DESCRIPTION OF EMBODIMENTS

The reasons for limiting the glass composition of the X-ray opaque glass of the present invention as indicated above will be described below. Note that, in the description regarding the content of each component of the glass, "%" means "mass%" unless otherwise indicated.

SiO₂ is a component that forms a glass network. It is also a component that has the effect of improving chemical durability and suppressing devitrification. The content of SiO₂ is preferably from 25 to 75%, from 30 to 70%, or from 40 to 65%, and is particularly preferably from 45 to 63%. When the SiO₂ content is too small, chemical durability tends to deteriorate; moreover, the glass tends to devitrify, which may make manufacturing difficult. Meanwhile, when the SiO₂ content is too large, meltability tends to decrease.

B₂O₃ is a component that forms a glass network. B₂O₃ is also a component that has an effect of improving chemical durability and suppressing devitrification with almost no increase in the refractive index. The content of B₂O₃ is preferably from 0 to 15% or from 0.5 to 10%, and is particularly preferably from 1 to 8%. When the B₂O₃ content is too large, meltability tends to decrease. In addition, B₂O₃ is a component that is relatively easily volatilized in a later-described flame polishing process for forming the glass filler into a spherical shape. Therefore, when the content of B₂O₃ is too large, volatilization in the spherical shape forming process increases, and it becomes difficult to obtain a glass filler having a desired composition.

Al₂O₃ is a vitrification stabilizing component. Al₂O₃ is also a component that has an effect of improving chemical durability and suppressing devitrification with almost no increase in the refractive index. The content of Al₂O₃ is preferably 5 to 23%, from 6 to 20%, from 6.4 to 18%, from 6.8 to 17%, from 7 to 16%, from 7.2 to 15%, from 7.4 to 15%, from 7.5 to 15%, from more than 7.5 to 15%, from 7.6 to 15%, or from 7.8 to 15%, and is particularly preferably from 8 to 15%. When the Al₂O₃ content is too small, chemical durability tends to decrease, and moreover, the glass tends to devitrify, which may make manufacturing difficult. Meanwhile, when the Al₂O₃ content is too large, meltability tends to decrease.

The ratio of Al₂O₃/B₂O₃ is preferably 0.6 or more, 0.8 or more, 1 or more, 1.2 or more, 1.5 or more, or 1.8 or more, and is particularly preferably 2 or more. In this case, when the glass filler is formed into a spherical shape by flame polishing, compositional variation due to volatilization of the glass component is less likely to occur. As a result, a substantially spherical glass filler having a desired composition can be easily obtained. The upper limit of the ratio of Al₂O₃/B₂O₃ is not particularly limited, but from the viewpoint of the ease of vitrification, the upper limit is preferably 100 or less, 50 or less, or 30 or less, and is particularly preferably 10 or less. Note that in the present specification, "x/y" means a value obtained by dividing the content of a component x by the content of a component y.

K₂O is a component that reduces the viscosity of glass and suppresses devitrification. The content of K₂O is preferably from 0 to 15%, from 0.1 to 14%, or from 0.5 to 13%, and is particularly preferably from 1 to 12%. When the content of K₂O is too large, chemical durability tends to decrease, and moreover, the glass tends to devitrify, which may make manufacturing difficult.

Cs₂O is a component having a high effect of improving X-ray opacity. In addition, similar to other alkali metal oxides, Cs₂O has an effect of reducing viscosity and suppressing devitrification. Also note that Cs₂O is a component that does not easily cause an increase in the refractive index. The content of Cs₂O is preferably from 0 to 30%, or from 5 to 27%, and is particularly preferably from 10 to 25%. When the content of the Cs₂O is too low, the effects described above are difficult to achieve. On the other hand, when the Cs₂O content is too large, chemical durability tends to decrease, and moreover, the glass tends to devitrify, which may make manufacturing difficult.

The X-ray opaque glass according to the present invention may contain a component below in addition to the components described above.

Similar to Cs₂O, BaO is a component having a high effect of improving X-ray opacity. As an intermediate substance, BaO also has an effect of stabilizing vitrification. The content of BaO is preferably from 0 to 20%, from 0.1 to 10%, from 0.3 to 6%, from 0.5 to less than 4%, or from 1 to 3%, and is particularly preferably from 1 to 2%. When the BaO content is too large, chemical durability tends to decrease, and moreover, the glass tends to devitrify, which may make manufacturing difficult. In addition, the refractive index tends to be unreasonably high.

SnO₂ is also a component having a high effect of improving X-ray opacity. Also note that SnO₂ is a component that does not easily increase the refractive index. The SnO₂ content is preferably from 0 to 20%, or from 0.1 to 10%, and is particularly preferably from 0.5 to 6%. If the content of SnO₂ is too large, the absorption edge wavelength of the glass is shifted, and thus an undesirable yellowish coloration occurs or the chemical durability is easily lowered. Moreover, the glass tends to devitrify, which may make manufacturing difficult.

La₂O₃ is also a component having a high effect of improving the X-ray opacity. The La₂O₃ content is preferably from 0 to 20%, or from 0.1% to 10%, and is particularly preferably from 0.5 to 6%. When the La₂O₃ content is too large, chemical durability tends to decrease, and moreover, the glass tends to devitrify, which may make manufacturing difficult. In addition, the refractive index tends to be unreasonably high.

In order to obtain desired X-ray opacity, the content of the Cs₂O + BaO + SnO₂ + La₂O₃ is preferably 10% or more, or 15% or more, and is particularly preferably 20% or more. However, when the Cs₂O + BaO + SnO₂ + La₂O₃ content is too large, chemical durability tends to decrease, and moreover, the glass tends to devitrify, which may make manufacturing difficult. In addition, the refractive index tends to be unreasonably high. Therefore, the upper limit of the Cs₂O + BaO + SnO₂ + La₂O₃ content is preferably 40% or less, or 30% or less, and is particularly preferably 25% or less. Note that the total amount of two or more components selected from Cs₂O, BaO, SnO₂, and La₂O₃ is also preferably within the above range.

Also note that since Cs₂O is less likely to increase the refractive index than the other components that improve the X-ray opacity, the preferential inclusion of Cs₂O makes it easier to achieve both the desired refractive index and the desired X-ray opacity. From such a viewpoint, Cs₂O/(Cs₂O + BaO + SnO₂ + La₂O₃) is preferably 0.2 or more, 0.3 or more, or 0.4 or more, and is particularly preferably 0.5 or more.

WO₃ also has an effect of improving X-ray opacity. In addition, WO₃ is a component that can adjust the refractive index and has an effect of reducing the viscosity of the glass. The WO₃ content is preferably from 0 to 6%, or from 0.1 to 5%, and is particularly preferably from 1 to 4.5%. When the WO₃ content is too large, the glass tends to devitrify.

Nb₂O₅ is a component that increases the refractive index. The content of Nb₂O₅ is preferably from 0 to 20%, from 0.1 to 15%, or from 0.5 to 10%, and is particularly preferably from 1 to 5%. When the Nb₂O₅ content is too large, the glass tends to devitrify.

ZrO₂ is a component that improves weather resistance and can adjust the refractive index. The content of ZrO₂ is preferably from 0% to 10%, or from 0% to 7.5%, and particularly preferably from 0% to 5%. When the ZrO₂ content is too large, the softening point tends to go up. In addition, devitrification resistance tends to decrease.

P₂O₅ is a component that forms a glass network and that improves the light transmittance and devitrification resistance of the glass. It is also a component that easily lowers the softening point of the glass. The content of P₂O₅ is preferably from 0 to 20%, from 0 to 15%, or from 0 to 10%, and is particularly preferably from 0.1 to 5%. When the P₂O₅ content is too large, the chemical durability easily decreases, and the refractive index tends to decrease. In addition, striae are likely to occur.

Li₂O is a component that reduces the viscosity of glass and suppresses devitrification. The content of the Li₂O is preferably from 0 to 10%, from 0.1 to 9%, or from 0.5 to 7%, and is particularly preferably from 1 to 5%. When the Li₂O content is too large, chemical durability tends to decrease, and moreover, the glass tends to devitrify, which may make manufacturing difficult.

Na₂O is a component that reduces the viscosity of glass and suppresses devitrification. The content of Na₂O is preferably from 0 to 20%, from 0.1 to 18%, or from 0.5 to 15%, and is particularly preferably from 1 to 10%. When the Na₂O content is too large, chemical durability tends to decrease, and moreover, the glass tends to devitrify, which may make manufacturing difficult.

Note that the content of R₂O (R is at least one kind selected from Li, Na, K, and Cs) is preferably from 5 to 50%, from 10 to 45%, or from 15 to 40%, and is particularly preferably from 20 to 35%. If the content of R₂O is too low, the effects described above are difficult to achieve. When the R₂O content is too large, chemical durability tends to decrease, and moreover, the glass tends to devitrify, which may make manufacturing difficult.

MgO, CaO, and SrO are components which, similar to BaO, stabilize vitrification as intermediate substances. In addition, they are components that easily reduce the viscosity of the glass without significantly reducing the chemical durability of the glass. The content of each of MgO, CaO, and SrO is preferably from 0 to 20%, from 0.1 to 15%, from 0.5 to 10%, from 1 to 9%, or from 1 to 5%, and is particularly preferably from 1 to 4%. When the content of these components is too large, chemical durability tends to deteriorate; moreover, the glass tends to devitrify, which may make manufacturing difficult.

Note that the content of R'O (R' is at least one kind selected from MgO, CaO, SrO, and BaO) is preferably from 0 to 20% or from 0.1 to 10%, and is particularly preferably from 0.5 to 6%. When the content of R'O is too large, chemical durability tends to decrease, and moreover, the glass tends to devitrify, which may make manufacturing difficult.

Similar to the R'O component, ZnO is a component that stabilizes vitrification as an intermediate substance. In addition, they are components that easily reduce the viscosity of the glass without significantly reducing the chemical durability of the glass. The content of ZnO is preferably from 0 to 20%, from 0.1 to 15%, from 0.5 to 10%, from 1 to 9%, or from 1 to 5%, and is particularly preferably from 1 to 4%. When the content of ZnO is too large, chemical durability tends to decrease, and moreover, the glass tends to devitrify, which may make manufacturing difficult.

NiO, Cr₂O₃, and CuO are components that color the glass and that easily reduce light transmittance, particularly in the ultraviolet region to the visible region. As such, the content of each of these components is preferably 1% or less, 0.75% or less, or 0.5% or less, and particularly preferably, these components are substantially not contained. Note that in the present specification, "substantially not contained" means that the component is not actively contained as a raw material, and does not exclude cases of unavoidable contamination by impurities. Objectively, this means that the content is less than 0.1%.

Sb₂O₃ and CeO₂ are components that tend to suppress a decrease in light transmittance. Moreover, when the contents of these components are too large, devitrification tends to occur. As such, the content of each of Sb₂O₃ and CeO₂ is preferably 1% or less, 0.8% or less, 0.5% or less, or 0.2% or less, and particularly preferably, Sb₂O₃ and CeO₂ are substantially not contained.

TiO₂ is a component that excessively increases the refractive index, colors the glass, and decreases the transmittance. Further, since TiO₂ has an X-ray opacity as low as that of ZnO or ZrO₂, even if TiO₂ is added, the TiO₂ increases the refractive index, and thereby makes it difficult to achieve both a low refractive index and a high X-ray opacity. Therefore, preferably, the glass of the present invention substantially does not contain TiO₂.

F is a component that can lower the melting temperature or adjust the refractive index. However, F is highly volatile, and therefore when producing a substantially spherical glass filler through flame polishing, for example, the volatilized component may adhere to the glass filler surface and deteriorate the surface properties. Moreover, the chemical durability of the glass tends to decrease when F is contained. Accordingly, the glass of the present invention is preferably substantially free of F.

For environmental reasons, the glass of the present invention contains substantially no Pb component (PbO or the like). Similarly, arsenic components (such as As₂O₃) are preferably substantially not contained for environmental reasons.

The X-ray opaque glass of the present invention is used, for example, as a particulate glass filler (glass beads). If the glass filler is substantially spherical, an increase in viscosity of the resin composition is easily suppressed by mixing the glass filler into a resin, and thus such a shape of the glass filler is preferable. Therefore, a large amount of the X-ray opaque glass filler can be contained in the resin composition, and as a result, the X-ray contrast property can be effectively enhanced. Note that, in addition to a particle shape, the X-ray opaque glass of the present invention may have a fiber shape or a bulk shape.

The average particle size of the glass filler is preferably from 0.01 to 50 µm, from 0.05 to 40 µm, from 0.1 to 40 µm, from 0.2 to 30 µm, from 0.3 to 20 µm, or from 0.5 to 10 µm, and is particularly preferably from 0.8 to 6 µm. This makes it easy to improve the surface smoothness of a formed body made of a cured product of the resin composition. When the average particle size of the glass particles is too small, the fluidity of the resin composition decreases, making it difficult for bubbles mixed in the resin composition to escape to the outside. Meanwhile, when the average particle size of the glass particles is too large, the curability of the resin composition tends to decrease. Here, the "average particle size" is a value measured by a laser diffraction device, and refers to a particle size at which the accumulated amount is 50% cumulatively from the smallest particle in a volume-based cumulative particle size distribution curve measured by the laser diffraction method.

In the X-ray opaque glass of the present invention, the aluminum equivalent thickness, which is an indicator of the X-ray opacity, and the refractive index nd satisfy a relationship of (aluminum equivalent thickness)/(refractive index nd) ≥ 1.6. In this way, the desired light transmission property and X-ray opacity can be achieved when the X-ray opaque glass is blended with a resin. The ratio of the (aluminum equivalent thickness)/(refractive index nd) is preferably 1.8 or more, 2 or more, or 2.2 or more, and is particularly preferably 2.5 or more. The upper limit is not particularly limited but is realistically 7 or less, 6 or less, or 5 or less.

The refractive index (nd) of the X-ray opaque glass of the present invention is, for example, from 1.48 to 1.58, preferably from 1.49 to 1.54, and particularly preferably from 1.50 to 1.52. In this way, the refractive index is easily matched with that of many curable resins such as acrylic resins and epoxy resins and a resin composition having excellent transparency or a formed body made of a cured product of the resin composition can be obtained more easily.

In the case of bulk glass, the refractive index of the X-ray opaque glass of the present invention can be measured using a V-block method-based precision refractometer. In addition, in the case of particulate (or fibrous) glass, the refractive index can be measured by the following method.

When the glass particles are suspended in a dispersion, the glass particles in the dispersion can be visually confirmed if the refractive index difference between the glass particles and the dispersion becomes large, but when the refractive indices of the two are matching, the glass particles cannot be visually confirmed and are seemingly transparent. Using this principle, the refractive index of the glass particles can be measured in the following manner.

When the refractive index of the dispersion is changed, the light transmittance of the dispersion alone and the light transmittance of the dispersion in which the glass particles are dispersed are measured. A correlation exists between the light transmittance difference between the two and the refractive index of the dispersion. Specifically, when the refractive index of the dispersion is plotted on the x-axis and the above light transmittance difference is plotted on the y-axis, a quadratic function having a minimum value is obtained. Here, the refractive index of the dispersion when the light transmittance difference is the minimum value can be regarded as the refractive index of the glass particles.

The dispersion is prepared, for example, by mixing two known types of liquids having refractive indices around the estimated refractive index of the glass particles. Since the refractive indices of the liquids are additive, a dispersion having a specific refractive index can be prepared by appropriately changing the mixing ratio of the two types of liquids.

When the light transmittance of the dispersion in which the glass particles are dispersed is to be measured, it is preferable to select a dispersion having a specific gravity as close as possible to the specific gravity of the glass particles in order to suppress the sedimentation of the glass particles. Alternatively, the addition amount of the glass particles is preferably increased such that the dispersion has a paste-like viscosity.

The glass of the present invention has an aluminum equivalent thickness, which is an indicator of X-ray opacity, of preferably 2.5 mm or more, 3 mm or more, or 3.5 mm or more, and particularly preferably 4 mm or more. The upper limit of the aluminum equivalent thickness is not particularly limited but is realistically 10 mm or less.

The surface of the glass filler is preferably silanized with a silane coupling agent or the like. Silanization can increase the bonding strength between the glass filler and a curable resin, and thereby a formed body having more excellent mechanical strength can be obtained. Furthermore, the compatibility between the glass filler and the curable resin is improved, and the generation of bubbles at the interface can be suppressed, and as a result, excessive light scattering can be suppressed. Preferred examples of the silane coupling agent include an aminosilane, an epoxysilane, and an acrylsilane.

The X-ray opaque glass of the present invention can be produced by melting and forming raw materials, and then subjecting the formed product to steps such as grinding and classification as necessary. The melting temperature is not particularly limited and may be a temperature at which the raw materials can be homogeneously melted. For example, the melting temperature is preferably from 1400°C to 1700°C, and particularly preferably from 1500°C to 1650°C.

For example, in a case of preparing a glass filler made of the X-ray opaque glass of the present invention, a molten glass is poured between a pair of cooling rollers and formed into a film shape, after which the resulting film-shaped formed body is ground to a predetermined size, and then classified, as necessary. Further, by flame polishing the obtained glass particles with an air burner or the like, the glass particles can be softened, fluidized, and formed into a substantially spherical shape.

### Resin composition

The resin composition of the present invention contains a curable resin and the above-mentioned glass filler. Specific examples of the curable resin will be described below.

An ultraviolet curable resin is preferably used as the curable resin. As the ultraviolet curable resin, a resin resulting from polymerization of radical species or cationic species is preferably used. For example, an acrylic resin, an epoxy resin or the like can be used. Examples of the acrylic resin include an ester acrylate resin and an urethane acrylate resin.

The acrylic resin may include the following compounds. For example, as a monofunctional compound, isobornyl acrylate, isobornyl methacrylate, dinclopentenyl acrylate, bornyl acrylate, bornyl methacrylate, 2-hydroxyethyl acrylate, 2-hydroxypropyl acrylate, propylene glycol acrylate, vinylpyrrolidone, acrylamide, vinyl acetate, styrene or the like are exemplified. As a polyfunctional compound, trimethylolpropane triacrylate, EO-modified trimethylolpropane triacrylate, ethylene glycol diacrylate, tetraethylene glycol diacrylate, polyethylene glycol diacrylate, 1,4-butanediol diacrylate, 1,6-hexanediol diacrylate, neopentyl glycol diacrylate, dicyclopentenyl diacrylate, polyester diacrylate, diallyl phthalate or the like are exemplified. These monofunctional compounds and polyfunctional compounds may be used alone or in combination of two or more types. However, these compounds are not limited to the description above.

For the acrylic resin, a photopolymerization initiator can be used as a polymerization initiator. Example of the polymerization initiator include 2,2-dimethoxy-2-phenylacetophenone, 1-hydroxycyclohexyl phenyl ketone, acetophenone, benzophenone, xanthone, fluorenone, benzaldehyde, fluorene, anthraquinone, triphenylamine, carbazole, 3-methylacetophenone, and Michler's ketone. These polymerization initiators may be used alone or in combination of two or more types. Each of these polymerization initiators is preferably contained, by mass%, in an amount of from 0.1 to 10% with respect to the monofunctional compound and the polyfunctional compound. As necessary, a sensitizer such as an aminebased compound may be used in combination.

The epoxy resin may include the following compounds. For example, hydrogenated bisphenol A diglycidyl ether, 3,4-epoxycyclohexylmethyl-3,4-epoxycyclohexane carboxylate, 2-(3,4-epoxycyclohexyl-5,5-spiro-3,4-epoxy)cyclohexane-m-dioxane, bis(3,4-epoxycyclohexylmethyl)adipate or the like are exemplified. When these compounds are used, an energy-activated cationic initiator such as triphenylsulfonium hexafluoroantimonate can be used.

Furthermore, a leveling agent, a surfactant, an organic polymer compound, an organic plasticizer, an anti-static agent, and the like may be added to the curable resin as necessary.

The content of the glass filler in the resin composition is preferably, by weight%, from 1 to 90%, from 5 to 80%, or from 10 to 70%, and is particularly preferably from 15 to 65%. When the content of the glass filler is too small, the mechanical strength of the formed body made of a cured product of the resin composition tends to decrease. Meanwhile, when the content of glass filler is too large, light scattering increases, and it becomes difficult to obtain a formed body having excellent transparency. In addition, the curability of the resin composition tends to decrease. Also, the viscosity of the curable resin tends to be too large, which may make handling difficult.

The difference between the refractive index nd of the glass filler and the refractive index nd of the curable resin before curing is preferably within ±0.1, within ±0.09, within ±0.08, or within ±0.07, and is particularly preferably within ±0.05. In this way, light scattering due to a difference between the refractive index of the curable resin and the refractive index of the glass filler in the stage of curing the resin composition can be suppressed.

The difference between the refractive index nd of the glass filler and the refractive index nd of the curable resin after curing is within ±0.1, preferably within ±0.08, within ±0.05, or within ±0.03, and particularly preferably within ±0.02. In this way, light scattering due to a difference between the refractive index of the resin after curing and the refractive index of the glass filler can be suppressed, and a formed body having excellent transparency can be easily obtained.

### Manufacturing method of formed body

Next, a manufacturing method of a formed body using the resin composition of the present invention will be described.

A formed body can be obtained by irradiating a light beam to the above-described resin composition of the present invention to cure the resin composition. Here, when an ultraviolet curable resin is used as the resin, ultraviolet rays may be used as the light beam in irradiation. For example, when the resin composition is used as a dental restorative material or the like (so-called dental composite resin), the treatment can be performed by applying the resin composition to a treatment site on a tooth and then irradiating a light beam to the resin composition to cure the resin composition.

### EXAMPLES

Hereinafter, the present invention will be described based on Examples, but the present invention is not limited to these Examples.

Tables 1 to 6 shows Examples (Nos. 1 to 22) of the present invention and Comparative Examples (Nos. 23 to 31).

**[Table 1]**

| [mass%] | 1 | 2 | 3 | 4 | 5 | 6 |
|---|---|---|---|---|---|---|
| SiO₂ | 56.6 | 61.6 | 60.1 | 58.6 | 31.1 | 61.6 |
| B₂O₃ | 6.9 | 2.0 | | | | 4.0 |
| Al₂O₃ | 8.0 | 8.0 | 8.0 | 8.0 | 18.9 | 8.0 |
| P₂O₅ | | | | | 15.0 | |
| Li₂O | 0.3 | 0.3 | | | | |
| Na₂O | 1.8 | 4.6 | 11.5 | 7.0 | 18.3 | 4.0 |
| K₂O | 0.7 | 0.9 | 1.1 | 8.1 | | 1.2 |
| Cs₂O | 18.4 | 15.0 | 10.0 | 10.0 | 12.7 | 15.0 |
| CaO | 0.2 | 0.2 | 0.1 | | | |
| SrO | 1.5 | 0.1 | 0.1 | | | |
| BaO | 1.4 | 1.0 | 5.6 | 4.6 | | |
| ZnO | 0.6 | 0.5 | 0.4 | | | 2.6 |
| Nb₂O₅ | | | | | | |
| WO₃ | 1.4 | 1.6 | | 0.7 | 4.0 | 1.6 |
| SnO₂ | 1.9 | 3.5 | 2.8 | 3.0 | | |
| La₂O₃ | 0.3 | 0.7 | 0.3 | | | 2.0 |
| Sb₂O₃ | | | | | | |
| SiO₂ + B₂O₃ + Al₂O₃ + P₂O₅ | 71.5 | 71.6 | 68.1 | 66.6 | 65.0 | 73.6 |
| Al₂O₃/B₂O₃ | 1.16 | 4.00 | - | - | - | 2.00 |
| R₂O | 21.2 | 20.8 | 22.6 | 25.1 | 31.0 | 20.2 |
| R'O | 3.1 | 1.3 | 5.8 | 4.6 | 0 | 0 |
| Cs₂O + BaO + SnO₂ + La₂O₃ | 22.0 | 20.2 | 18.7 | 17.6 | 12.7 | 17.0 |
| Cs₂O/(Cs₂O + BaO + SnO₂ + La₂O₃) | 0.84 | 0.74 | 0.53 | 0.57 | 1.00 | 0.88 |
| Refractive index nd | 1.507 | 1.510 | 1.516 | 1.515 | 1.501 | 1.505 |
| Density [g/cm³] | 2.661 | 2.668 | 2.687 | 2.686 | 2.663 | 2.608 |
| Al equivalent thickness [mm] | 4.5 | 4.0 | 3.5 | 3.6 | 2.9 | 3.4 |
| (Al equivalent thickness)/(refractive index nd) | 2.99 | 2.65 | 2.31 | 2.38 | 1.93 | 2.26 |
| Chemical durability | Good | Good | Good | Good | Good | Good |

**[Table 2]**

| [mass%] | 7 | 8 | 9 | 10 | 11 | 12 |
|---|---|---|---|---|---|---|
| SiO₂ | 56.6 | 50.0 | 56.7 | 55.3 | 54.6 | 53.9 |
| B₂O₃ | 4.0 | 6.0 | 3.0 | 2.4 | 1.8 | 1.2 |
| Al₂O₃ | 8.0 | 10.0 | 8.0 | 8.0 | 8.0 | 8.0 |
| P₂O₅ | | | | | | |
| Li₂O | | | | | | |
| Na₂O | 5.0 | | | | | |
| K₂O | 1.2 | 9.0 | 5.0 | 8.0 | 10.0 | 12.0 |
| Cs₂O | 20.0 | 20.0 | 21.3 | 21.0 | 20.7 | 20.4 |
| CaO | | | 1.5 | 1.3 | 1.1 | 0.6 |
| SrO | | | 2.5 | 2.7 | 2.9 | 3.4 |
| BaO | | | | | | |
| ZnO | 2.6 | | 1.3 | 0.8 | 0.5 | 0.3 |
| Nb₂O₅ | | 1.3 | | | | |
| WO₃ | 1.6 | 2.1 | | | | |
| SnO₂ | | | | | | |
| La₂O₃ | 1.0 | 1.3 | 0.7 | 0.5 | 0.4 | 0.2 |
| Sb₂O₃ | | 0.3 | | | | |
| SiO₂ + B₂O₃ + Al₂O₃ + P₂O₅ | 68.6 | 66.0 | 67.7 | 65.7 | 64.4 | 63.1 |
| Al₂O₃/B₂O₃ | 2.00 | 1.67 | 2.67 | 3.33 | 4.44 | 6.67 |
| R₂O | 26.2 | 29.0 | 26.3 | 29.0 | 30.7 | 32.4 |
| R'O | 0 | 0 | 4.0 | 4.0 | 4.0 | 4.0 |
| Cs₂O + BaO + SnO₂ + La₂O₃ | 21.0 | 21.3 | 22.0 | 21.5 | 21.1 | 20.6 |
| Cs₂O/(Cs₂O + BaO + SnO₂ + La₂O₃) | 0.95 | 0.94 | 0.97 | 0.98 | 0.98 | 0.99 |
| Refractive index nd | 1.512 | 1.510 | 1.504 | 1.508 | 1.512 | 1.513 |
| Density [g/cm³] | 2.718 | 2.635 | 2.618 | 2.700 | 2.696 | 2.712 |
| Al equivalent thickness [mm] | 4.4 | 3.8 | 4.7 | 4.6 | 4.8 | 4.8 |
| (Al equivalent thickness)/(refractive index nd) | 2.91 | 2.52 | 3.13 | 3.05 | 3.17 | 3.17 |
| Chemical durability | Good | Good | Good | Good | Good | Good |

**[Table 3]**

| [mass%] | 13 | 14 | 15 | 16 | 17 | 18 |
|---|---|---|---|---|---|---|
| SiO₂ | 51.5 | 51.7 | 50.0 | 48.9 | 47.6 | 51.2 |
| B₂O₃ | 4.0 | 4.0 | 8.0 | 9.0 | 10.0 | 4.9 |
| Al₂O₃ | 8.0 | 8.0 | 8.0 | 8.0 | 8.0 | 8.5 |
| P₂O₅ | | | | | | |
| Li₂O | | | | | | |
| Na₂O | 2.0 | 2.0 | | | | |
| K₂O | 8.0 | 8.0 | 8.0 | 8.0 | 8.5 | 8.6 |
| Cs₂O | 22.8 | 22.7 | 22.5 | 22.8 | 23.0 | 23.4 |
| CaO | 3.0 | 3.0 | 1.0 | 1.0 | 1.0 | 2.4 |
| SrO | | | | | | |
| BaO | | | | | | |
| ZnO | 0.7 | | | | | |
| Nb₂O₅ | | | | | | |
| WO₃ | | 0.6 | 1.5 | 1.3 | 0.9 | |
| SnO₂ | | | 1.0 | 1.0 | 1.0 | |
| La₂O₃ | | | | | | 1.0 |
| Sb₂O₃ | | | | | | |
| SiO₂ + B₂O₃ + Al₂O₃ + P₂O₅ | 63.5 | 63.7 | 66.0 | 65.9 | 65.6 | 64.6 |
| Al₂O₃/B₂O₃ | 2.00 | 2.00 | 1.00 | 0.89 | 0.80 | 1.73 |
| R₂O | 32.8 | 32.7 | 30.5 | 30.8 | 31.5 | 32.0 |
| R'O | 3.0 | 3.0 | 1.0 | 1.0 | 1.0 | 2.4 |
| Cs₂O + BaO + SnO₂ + La₂O₃ | 22.8 | 22.7 | 23.5 | 23.8 | 24.0 | 24.4 |
| Cs₂O/(Cs₂O + BaO + SnO₂ + La₂O₃) | 1.00 | 1.00 | 0.96 | 0.96 | 0.96 | 0.96 |
| Refractive index nd | 1.508 | 1.508 | 1.511 | 1.509 | 1.508 | 1.511 |
| Density [g/cm³] | 2.672 | 2.664 | 2.673 | 2.651 | 2.642 | 2.712 |
| Al equivalent thickness [mm] | 3.9 | 3.8 | 4.2 | 4.0 | 3.7 | 4.5 |
| (Al equivalent thickness)/(refractive index nd) | 2.59 | 2.52 | 2.78 | 2.65 | 2.45 | 2.98 |
| Chemical durability | Good | Good | Good | Good | Good | Good |

**[Table 4]**

| [mass%] | 19 | 20 | 21 | 22 |
|---|---|---|---|---|
| SiO₂ | 52.8 | 51.6 | 71.6 | 70.2 |
| B₂O₃ | 5.0 | 4.0 | 1.0 | 1.0 |
| Al₂O₃ | 8.5 | 8.0 | 6.0 | 6.0 |
| P₂O₅ | | | | |
| Li₂O | | | | |
| Na₂O | | 5.0 | | |
| K₂O | 8.7 | 1.2 | 0.8 | 1.0 |
| Cs₂O | 21.6 | 25.0 | | |
| CaO | 1.4 | | | |
| BaO | | 2.6 | 15.0 | 15.0 |
| ZnO | | 2.6 | | |
| Nb₂O₅ | | | | |
| WO₃ | | | | |
| SnO₂ | | | 4.5 | 2.0 |
| La₂O₃ | 2.0 | | 1.1 | 4.8 |
| NaF | | | | |
| SiO₂ + B₂O₃ + Al₂O₃ + P₂O₅ | 66.3 | 63.6 | 78.6 | 77.2 |
| Al₂O₃/B₂O₃ | 1.70 | 2.00 | 6.00 | 6.00 |
| R₂O | 30.3 | 31.2 | 0.8 | 1.0 |
| R'O | 1.4 | 2.6 | 15.0 | 15.0 |
| Cs₂O + BaO + SnO₂ + La₂O₃ | 23.6 | 27.6 | 20.6 | 21.8 |
| Cs₂O/(Cs₂O + BaO + SnO₂ + La₂O₃) | 0.92 | 0.91 | 0.00 | 0.00 |
| Refractive index nd | 1.509 | 1.520 | 1.520 | 1.531 |
| Density [g/cm³] | 2.704 | 2.800 | 2.681 | 2.759 |
| Al equivalent thickness [mm] | 4.4 | 5.6 | 2.5 | 2.6 |
| (Al equivalent thickness)/(refractive index nd) | 2.92 | 3.68 | 1.64 | 1.68 |
| Chemical durability | Good | Good | Good | Good |

**[Table 5]**

| [mass%] | 23 | 24 | 25 | 26 | 27 | 28 |
|---|---|---|---|---|---|---|
| SiO₂ | 52.9 | 50.0 | 50.0 | 50.0 | 50.0 | 48.0 |
| B₂O₃ | 15.8 | 20.0 | 20.0 | 20.0 | 20.0 | 20.0 |
| Al₂O₃ | 16.0 | | | | | 2.0 |
| P₂O₅ | | | | | | |
| Li₂O | | | 0.1 | 0.5 | 0.1 | 1.1 |
| Na₂O | | | 3.4 | 8.0 | 8.6 | 7.6 |
| K₂O | 3.4 | 20.0 | 15.0 | 10.0 | 10.0 | 9.3 |
| Cs₂O | | | | | | |
| CaO | 1.5 | | | | | |
| SrO | | | | | | |
| BaO | | | | | | |
| TiO2 | 1.2 | | | | | |
| ZnO | 1.5 | | | | | |
| ZrO2 | | 3.5 | 5.8 | 2.7 | 3.0 | 4.0 |
| Nb₂O₅ | 3.7 | | | | | |
| WO₃ | 4.0 | | | | | |
| SnO₂ | | | | 5.0 | 4.0 | |
| La₂O₃ | | 6.5 | 5.7 | 3.8 | 4.3 | |
| Yb₂O₃ | | | | | | 8.0 |
| NaF | | | | | | |
| SiO₂ + B₂O₃ + Al₂O₃ + P₂O₅ | 84.7 | 70.0 | 70.0 | 70.0 | 70.0 | 70.0 |
| Al₂O₃/B₂O₃ | 1.01 | 0.00 | 0.00 | 0.00 | 0.00 | 0.10 |
| R₂O | 3.4 | 20.0 | 18.5 | 18.5 | 18.7 | 18.0 |
| R'O | 1.5 | 0 | 0 | 0 | 0 | 0 |
| Cs₂O + BaO + SnO₂ + La₂O₃ | 0 | 6.5 | 5.7 | 8.8 | 8.3 | 0 |
| Cs₂O/(Cs₂O + BaO + SnO₂ + La₂O₃) | - | 0.00 | 0.00 | 0.00 | 0.00 | - |
| Refractive index nd | 1.514 | 1.529 | 1.536 | 1.539 | 1.538 | 1.536 |
| Density [g/cm³] | 2.385 | 2.561 | 2.592 | 2.641 | 2.638 | 2.652 |
| Al equivalent thickness [mm] | 1.6 | 2.2 | 2.4 | 2.4 | 2.3 | 2.4 |
| (Al equivalent thickness)/(refractive index nd) | 1.06 | 1.44 | 1.56 | 1.56 | 1.50 | 1.56 |
| Chemical durability | Good | Poor | Poor | Poor | Poor | Poor |

**[Table 6]**

| [mass%] | 29 | 30 | 31 |
|---|---|---|---|
| SiO₂ | 49.7 | 53.0 | 51.2 |
| B₂O₃ | 20.0 | 17.0 | 3.2 |
| Al₂O₃ | 4.0 | | |
| P₂O₅ | | | |
| Li₂O | 1.8 | | |
| Na₂O | 9.0 | 10.5 | |
| K₂O | 7.4 | 8.6 | 10.0 |
| Cs₂O | | | 30.0 |
| CaO | | 2.3 | |
| SrO | | | |
| BaO | | | 5.6 |
| TiO2 | | | |
| ZnO | | | |
| ZrO₂ | | | |
| Nb₂O₅ | | | |
| WO₃ | | | |
| SnO₂ | | | |
| La₂O₃ | | | |
| Yb₂O₃ | 8.1 | 8.6 | |
| NaF | | | |
| SiO₂ + B₂O₃ + Al₂O₃ + P₂O₅ | 73.7 | 70.0 | 54.4 |
| Al₂O₃/B₂O₃ | 0.20 | 0.00 | 0.00 |
| R₂O | 18.2 | 19.1 | 40.0 |
| R'O | 0 | 2.3 | 5.6 |
| Cs₂O + BaO + SnO₂ + La₂O₃ | 0 | 0 | 35.6 |
| Cs₂O/(Cs₂O + BaO + SnO₂ + La₂O₃) | - | - | 0.73 |
| Refractive index nd | 1.528 | 1.533 | 1.527 |
| Density [g/cm³] | 2.607 | 2.679 | 2.925 |
| Al equivalent thickness [mm] | 1.8 | 2.0 | 7.2 |
| (Al equivalent thickness)/(refractive index nd) | 1.18 | 1.30 | 4.72 |
| Chemical durability | Poor | Poor | Poor |

Raw material powders were prepared and uniformly mixed so as to have the compositions described in the tables. Each resulting raw material batch was melted at 1450 to 1650°C until the batch became homogeneous, after which the molten raw material batch was formed, and thereby a glass was obtained.

The refractive index nd, density, aluminum equivalent thickness (X-ray contrast property), and chemical durability of each obtained glass were evaluated as described below.

The refractive index was measured using a precision refractometer (PR-2000 available from Kalnew).

The density was measured by the Archimedes method.

The aluminum equivalent thickness of a sample processed to a thickness of 1.0 mm was measured by a method based on JIS T6514:2015 using a digital X-ray device. Specifically, a gray scale value obtained by a digital X-ray device was processed by image analysis software to determine the X-ray absorbing aluminum equivalent thickness.

The chemical durability was evaluated through a HAST test. More specifically, each sample was held for 24 hours at conditions including a temperature of 21°C, a humidity of 95%, and an atmospheric pressure of 2 atm using the PC-242HSR2 HAST tester available from Hirayama Manufacturing Corporation, after which the surface of the sample was visually observed. If no change in appearance was found, the sample was evaluated as being "good", and if a change in appearance was observed, the sample was evaluated as being "poor".

As shown in Tables 1 to 4, the glasses of Examples Nos. 1 to 22 exhibited desired characteristics in which the ratio of the (aluminum equivalent thickness)/(refractive index nd) was from 1.64 to 3.68. More specifically, the refractive indices nd were from 1.501 to 1.531, which were desired refractive indices, and the aluminum equivalent thickness values ranged from 2.5 to 5.6 mm, and the X-ray opacity was excellent. Examples Nos. 1 to 22 also exhibited excellent chemical durability.

On the other hand, as shown in Tables 5 and 6, with the glasses of Nos. 23 to 30, the ratio of the (aluminum equivalent thickness)/(refractive index nd) was small with values of 1.56 or less, and these glasses did not exhibit desired properties. In addition, the glasses of Nos. 24 to 31 were inferior in chemical durability.

### INDUSTRIAL APPLICABILITY

The X-ray opaque glass, glass filler, and resin composition of the present invention are suitable for dental composite resins, cores, bonds, dental crown fillers, glass ionomer cements, and the like.

## Claims

1. An X-ray opaque glass comprising, by mass%, from 25 to 75% of SiO₂, from 0 to 15% of B₂O₃, from 5 to 23% of Al₂O₃, from 0 to 15% of K₂O, and from 0 to 30% of Cs₂O, and substantially not comprising a Pb component,
the X-ray opaque glass having an aluminum equivalent thickness, which is an indicator of X-ray opacity, and a refractive index nd satisfying a relationship of (aluminum equivalent thickness)/(refractive index nd) ≥ 1.6.

2. An X-ray opaque glass comprising, by mass%, from 25 to 75% of SiO₂, from 0 to 15% of B₂O₃, from 5 to 23% of Al₂O₃, from 0 to 15% of K₂O, from 0 to 30% of Cs₂O, and from 10 to 40% of Cs₂O + BaO + SnO₂ + La₂O₃, and substantially not comprising a Pb component.

3. The X-ray opaque glass according to claim 1 or 2, wherein a refractive index nd is from 1.48 to 1.58.

4. The X-ray opaque glass according to claim 1 or 2, wherein an X-ray opacity is 2.5 mm or more in terms of an aluminum equivalent thickness.

5. The X-ray opaque glass according to claim 1 or 2, substantially not comprising an F component.

6. The X-ray opaque glass according to claim 1, comprising, by mass%, 10% or more of Cs₂O + BaO + SnO₂ + La₂O₃.

7. The X-ray opaque glass according to claim 1 or 2, substantially not comprising TiO₂.

8. The X-ray opaque glass according to claim 1 or 2, comprising, by mass%, from 0 to 6% of WO₃.

9. A glass filler made of the X-ray opaque glass according to claim 1 or 2.

10. The glass filler according to claim 9, wherein the glass filler is substantially spherical.

11. The glass filler according to claim 10, having an average particle size of from 0.01 to 50 µm.

12. The glass filler according to claim 10, wherein a surface of the glass filler is silanized.

13. A resin composition comprising the glass filler according to claim 9 and a curable resin.

14. The resin composition according to claim 13, comprising, by weight%, from 1 to 90% of the glass filler.

15. The resin composition according to claim 13, the resin composition being for dental use.
